# EUROPEAN PATENT SPECIFICATION

(11) **EP 3 287 447 B1**
(45) Date of publication and mention of the grant of the patent: **11.11.2026**
(21) Application number: 17191453.4
(22) Date of filing: 22.07.2011
(51) Int. Cl.: C07D 275/06, C07D 291/06, C07C 215/54, A61K 31/137, C07D 239/545, C07C 59/255, A61P 25/04, A61K 31/455

(54) **SALTS OR CO-CRYSTALS OF 3-(3-DIMETHYLAMINO-1-ETHYL-2-METHYL-PROPYL)-PHENOL**
SALZE ODER KOKRISTALLE AUS 3-(3-DIMETHYLAMINO-1-ETHYL-2-METHYLPROPYL)PHENOL
SELS OU CO-CRISTAUX DE 3-(3-DIMETHYLAMINO-1-ETHYL-2-METHYL-PROPYL)- PHÉNOL

(30) Priority: 23.07.2010 EP 10007672
(43) Date of publication of application: 28.02.2018
(60) Divisional application: 19191640.2 / 3 597 628; 19206638.9 / 3 636 629; 19214270.1 / 3 650 439; 19214275.0 / 3 656 765; 19215642.0 / 3 653 600; 20204993.8 / 3 800 179
(62) Document number(s) of the earlier application(s) in accordance with Art. 76 EPC: 11743775.6 / 2 595 971
(73) Proprietor: Grünenthal GmbH, 52078 Aachen (DE)
(72) Inventor: GRUSS, Michael, 52062 Aachen (DE); KRASZEWSKI, Magda, 52070 Aachen (DE)
(74) Representative: Kutzenberger Wolff & Partner

(56) References cited:
- EP-A1- 0 693 475
- EP-A1- 1 612 203
- WO-A1-2007/144005
- WO-A1-2008/110323
- WO-A1-2012/051246
- WO-A2-02/067651
- WO-A2-2009/067703
- US-A1- 2005 176 790
- US-A1- 2008 269 326
- US-B2- 7 569 610
- US-E- R E39 593

## Description

The invention relates to a medicament comprising a salt or cocrystal of (1R,2R)-3-(3-dimethylamino-1-ethyl-2-methyl-propyl)-phenol (component a) and acid component (b2) being hydrogen bromide, wherein the salt or cocrystal of component (a) and component (b2) is present in crystalline and/or amorphous form, for use in the treatment of pain. The invention is set out in the appended claims.

3-(3-Dimethylamino-1-ethyl-2-methyl-propyl)-phenol compounds such as e.g. (1R,2R)-3-(3-dimethylamino-1-ethyl-2-methyl-propyl)-phenol, which is also known as tapentadol (CAS no. 175591-23-8) are synthetic, centrally acting analgesics which are effective in the treatment of pain.

Tapentadol exhibits a dual mechanism of action, on the one hand as a µ-opioid receptor agonist and on the other as a noradrenaline transporter inhibitor. In humans, the affinity of tapentadol to the recombinantly produced µ-opioid receptor is 18-times less than that of morphine. However, clinical studies have shown the pain-alleviating action of tapentadol to be only two to three times less than that of morphine. The only slightly reduced analgesic efficacy with a simultaneously 18-times reduced affinity to the recombinant µ-opioid receptor indicates that the noradrenaline transporter inhibiting property of tapentadol also contributes to its analgesic efficacy. Consequently, it may be assumed that tapentadol has a similar analgesic efficacy to that of pure µ-opioid receptor agonists but has fewer of the side effects associated with the µ-opioid receptor. The compound can be used in the form of its free base or as a salt or solvate. The production of the free tapentadol base and its hydrochloride salt are known for example from EP-A 0 693 475.

Conventional formulations for oral administration of a 3-(3-dimethylamino-1-ethyl-2-methylpropyl)-phenol compound such as tapentadol usually lead to rapid release of the active substance in the gastrointestinal tract, thereby leading to a rather rapid onset of its analgesic action. Subsequently, a rather rapid reduction in the action is observed. In order to achieve an effective analgesic action over a prolonged period of time, i.e. to ensure an adequately high concentration of the active substance in the patient's blood plasma, it is therefore necessary to administer the pharmaceutical composition comprising said active substance at relatively short time intervals. However, the need for frequent dosing may lead to errors in administration and to undesirable variations in the concentration of the compound in the blood plasma which could be detrimental to patient compliance and the therapeutic benefit, particularly when treating chronically painful conditions.

In order to overcome such disadvantages of conventional formulations, EP-A 1 439 829 suggests providing a delayed-release pharmaceutical composition suitable for oral administration comprising 3-(3-dimethylamino-1-ethyl-2-methyl-propyl)-phenol or its hydrogen chloride salt by means of retardation, i.e. by means of a matrix, a coating or in a release system based on osmotic action. Since the hydrogen chloride salt of 3-(3-dimethylamino-1-ethyl-2-methyl-propyl)-phenol has a high solubility in water and aqueous media, the manufacture of such a delayed-release formulation by retardation is considered necessary in order to achieve a controlled release of 3-(3-dimethylamino-1-ethyl-2-methylpropyl)-phenol subsequent to administration.

However, there is still a need for alternative administration forms comprising a 3-(3-dimethylamino-1-ethyl-2-methyl-propyl)-phenol compound such as tapentadol by which a controlled release of the active substance can be achieved. Further, there is a need for such administration forms which do not necessarily have to be formulated using additional means of retardation.

It was therefore an object of the present invention to provide a 3-(3-dimethylamino-1-ethyl-2-methyl-propyl)-phenol compound in a form suitable for administration, in particular in a solid form, e.g. for oral administration, which has advantages over the prior art, for example with respect to solubility, absorbability, concentration in the blood, or bioavailability of the pharmacologically active compound.

Another object of the present invention was to provide a 3-(3-dimethylamino-1-ethyl-2-methyl-propyl)-phenol compound in a form suitable for administration, e.g. in a solid form, which allows for a controlled release of the 3-(3-dimethylamino-1-ethyl-2-methyl-propyl)-phenol compound, without having necessarily to be present in a delayed-release form by retardation as e.g. described in EP-A 1 439 829 or by depot formulation.

These objects have been achieved by the pharmaceutical salts or co-crystals according to the present invention.

It has been surprisingly found that a 3-(3-dimethylamino-1-ethyl-2-methyl-propyl)phenol compound as component (a) in the form of a salt or cocrystal with at least one acid (b2) according to the present invention allows for the control of solubility, preferably of solubility in an aqueous medium such as water, of said salt or cocrystal and, thus of its component (a). This facilitates release in a targeted manner.

Particularly, it has been surprisingly found that said control of the solubility of component (a) can be influenced by the solubility of the inventive salt or cocrystal, i.e. by the choice of the acid (b2), e.g. by employing the acid (b2) for the formation of the inventive salt or cocrystal, which is less soluble in an aqueous medium such as water than for instance a hydrochloric acid addition salt (but which still has a sufficient or good solubility in said medium necessary for the desired form of administration such as oral administration), thereby leading to a different solubility profile, in particular a reduced solubility profile, and thus to a different pharmacokinetic profile of the resulting salt or cocrystal and, thus, to a controlled release profile of its component (a).

Therefore, the inventive salt or cocrystal allows for a controlled release of the 3-(3-dimethylamino-1-ethyl-2-methyl-propyl)phenol compound due to an intrinsic property of said salt or cocrystal by choice of the at least one suitable acid (b2). Thus, in contrast to conventional formulations comprising 3-(3-dimethylamino-1-ethyl-2-methyl-propyl)phenol compound, e.g. a formulation comprising the highly water-soluble hydrochloric acid addition salt of tapentadol, the inventive salts or cocrystals do not necessarily have to be provided in form of a delayed release form by means of a matrix, a coating or in a release system displaying an osmotic action as described in EP-A 1 439 829, or in a depot formulation in order to achieve the desired controlled release properties.

Thus, in one of its aspects the present invention relates to a medicament comprising at least one salt or cocrystal of (a) (1R,2R)-3-(3-dimethylamino-1-ethyl-2-methyl-propyl)-phenol, and (b2) hydrogen bromide, wherein the salt or cocrystal of component (a) and component (b2) is present in crystalline and/or amorphous form for use in the treatment of pain.

According to the disclosure, the 3-(3-dimethylamino-1-ethyl-2-methyl-propyl)-phenol compound, i.e. component (a) of the inventive salt or cocrystal, is a compound according to formulae (**I-a**), (**I-b**), (**I-c**) or (**I-d**) or any mixture in any mixing ratio thereof: 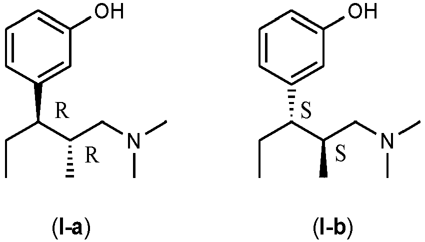 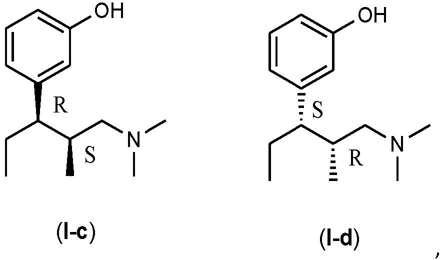 namely a compound selected from the group consisting of
(1R,2R)-3-(3-dimethylamino-1-ethyl-2-methyl-propyl)-phenol (**I-a**),
(1S,2S)-3-(3-dimethylamino-1-ethyl-2-methyl-propyl)-phenol (**I-b**),
(1R,2S)-3-(3-dimethylamino-1-ethyl-2-methyl-propyl)-phenol (**I-c**)
(1S,2R)-3-(3-dimethylamino-1-ethyl-2-methyl-propyl)-phenol (**I-d**),
and any mixture thereof.

The inventive compound is the compound according to formula (**I-a**), i.e. is (1R,2R)-3-(3-dimethylamino-1-ethyl-2-methyl-propyl)-phenol (**I-a**). As stated above the compound according to formula (**I-a**) is also known as tapentadol.

The present invention relates to a salt or cocrystal of (a) (1R,2R)-3-(3-dimethylamino-1-ethyl-2-methyl-propyl)-phenol and (b2) hydrogen bromide acid.

The inventive salt or cocrystal formed from component (a) and component (b2) is preferably present in crystalline or amorphous form, more preferably the inventive salt or cocrystal formed from component (a) and component (b2) is crystalline.

The salts or cocrystals according to the present invention may also be present in form of mixture with known salts of tapentadol such as tapentadol hydrochloride. Moreover, such known tapentadol compounds may also be present to form a co-crystal.

In a preferred embodiment the inventive salts or cocrystals of 3-(3-dimethylamino-1-ethyl-2-methyl-propyl)-phenol are physiologically acceptable salts or cocrystals, e.g. physiologically acceptable acid addition salts or cocrystals. For the purpose of the specification, the term "physiologically acceptable salt" or "physiologically acceptable cocrystal" refers to a salt or cocrystal of a 3-(3-dimethylamino-1-ethyl-2-methyl-propyl)-phenol compound and at least acid (b2), that are physiologically acceptable if administered to a mammal, preferably a human.

The inventively claimed salt or cocrystal may be in anhydrous and/or essentially solvent-free form, or be in hydrate and/or solvate form.

The acid according to component (b2) employed for the formation of the inventive salt or cocrystal may be in each case optionally be employed in the form of a solvate, in particular a hydrate.

The salts or cocrystals according to the present invention may contain the components (a) and (b2) in different ratios as is clear to those skilled in the art.

In a preferred embodiment of the present invention the salt or cocrystal may comprise component (a) and component (b2), respectively, within a ratio from 1:0.4 to 1:2.1, more preferably within the range of from 1:0.4 to 1:1.1.

Some of the inventively claimed salts or cocrystals may be present as so-called hemi-salts or hemi-cocrystals, i.e. the components (a) and (b2) are essentially present in a 2:1-stochiometry. Such hemi salts or hemi-cocrystals are well known to those skilled in the art.

In general, the solubility of the inventive salt or cocrystal may not only be influenced in a targeted manner by the choice of the acid (b2), but further may be additionally influenced by the solid state of said salt or cocrystal, e.g. whether the salt or cocrystal is present in a crystalline form such as the inventive salt or cocrystal formed from component (a) and component (b2), or whether the salt or cocrystal is present in an amorphous form.

Solid forms of the inventive salts or cocrystals may have a plurality of different internal structures and physicochemical properties (crystal polymorphism or polyamorphism) depending on the reaction conditions and/or crystallization/co-crystallization conditions employed for the synthesis of the salt or cocrystal. Therefore, crystalline forms of the inventive salt or cocrystal encompass such individual crystals and/or mixtures thereof in any ratio as a crystalline solid. Further, solid forms of the inventive salt or cocrystal may be mixtures of a crystalline and/or amorphous form of the salt or cocrystal, i.e. a crystalline and/or an amorphous form of the salt or cocrystal. Preferably, however, solid forms of the inventive salt or cocrystal are crystalline do not comprise an amorphous form.

A person skilled in the art is aware of the techniques which can be employed to obtain and identify the crystalline and/or amorphous forms of the inventive salts or cocrystals. In general, crystals and co-crystals are solids having an internal structure which is three-dimensionally formed by regular repetition of constituent atoms or groups of constituent atoms. In contrast, an amorphous solid does not have such a regular internal structure. A person skilled in the art is aware that e.g. powder X-ray diffraction giving specific X-ray powder diffraction patterns ("XRPD") or differential scanning calorimetry are suitable methods which can be employed in order to find out whether a solid is a crystalline or amorphous or any mixture thereof. For example, in X-ray powder diffraction of a solid using X-rays, the solid is determined to be a crystalline or partly crystalline when at least a specific peak is observed in its X-ray powder diffraction pattern ("XRPD"), and the solid is determined to be amorphous when no specific peak is observed. X-ray single crystal diffraction technique may be used for determination whether a compound forms a salt or a cocrystal or any mixture thereof.

It has been found that under suitable conditions some of the inventive salts or cocrystals can be obtained in the form of different polymorphs.

These crystalline forms make it possible to obtain the respective salts or cocrystals with high yields and high purity. These forms are further distinguished in that they are very easy to handle and allow an exact metering of the active ingredient.

Moreover, different polymorphs of said pharmaceutical active ingredient have fundamentally different properties, which may provide further advantages.

On the one hand, the advantages may be based on a particular physical property of a particular modification, for example, in relation to the handling or storage thereof, for example thermodynamic stability; crystal morphology, in particular structure, size, colour; density; bulk density; hardness; deformability; calorimetric characteristics, in particular melting point; solubility properties, in particular intrinsic rate of dissolution and equilibrium solubility; hygroscopicity; relative moisture profile; adhesion, etc.

On the other hand, the crystalline modification may also have improved chemical properties. For example, better thermodynamic stability can lead to improved chemical stability and longer storage life for chemical compounds, in particular compared to metastable and/or amorphous forms.

Another subject-matter of the present invention relates to a crystalline modification of the salt or cocrystal of hydrobromic acid and (1R,2R)-3-(3-dimethylamino-1-ethyl-2-methyl-propyl)-phenol.

This crystalline modification of the salt or cocrystal of hydrobromic acid and (1R,2R)-3-(3-dimethylamino-1-ethyl-2-methyl-propyl)-phenol according to the invention has at least one or more X-ray diffraction peaks selected from the group consisting of 9.93±0.20 (2Θ), 14.39±0.20 (2Θ), 15.22±0.20 (2Θ), 17.60±0.20 (2Θ), 20.81±0.20 (2Θ), 21.61±0.20 (2Θ), 24.37±0.20 (2Θ), 24.73±0.20 (2Θ), 25.19±0.20 (2Θ), 27.14±0.20 (2Θ), 27.94±0.20 (2Θ), 29.00±0.20 (2Θ) and 30.75±0.20 (2Θ).

Preferably, the crystalline modification of the salt or cocrystal of hydrobromic acid and (1R,2R)-3-(3-dimethylamino-1-ethyl-2-methyl-propyl)-phenol according to the invention may additionally have at least one X-ray diffraction peak selected from the group consisting of 16.60±0.20 (2Θ), 19.48±0.20 (2Θ), 19.95±0.20 (2Θ), 21.26±0.20 (2Θ), 25.52±0.20 (2Θ), 25.69±0.20 (2Θ), 26.05±0.20 (2Θ), 29.44±0.20 (2Θ), 29.67±0.20 (2Θ), 30.13±0.20 (2Θ), 32.25±0.20 (2Θ), 33.02±0.20 (2Θ), 33.48±0.20 (2Θ), and 38.69±0.20 (2Θ).

The crystalline modification of the salt or cocrystal of hydrobromic acid and (1R,2R)-3-(3-dimethylamino-1-ethyl-2-methyl-propyl)-phenol according to the invention may further be characterised in that as well as one or more X-ray diffraction peaks selected from the group consisting of 9.93±0.20 (2Θ), 14.39±0.20 (2Θ), 15.22±0.20 (2Θ), 17.60±0.20 (2Θ), 20.81±0.20 (2Θ), 21.61±0.20 (2Θ), 24.37±0.20 (2Θ), 24.73±0.20 (2Θ), 25.19±0.20 (2Θ), 27.14±0.20 (2Θ), 27.94±0.20 (2Θ), 29.00±0.20 (2Θ) and 30.75±0.20 (2Θ) and optionally one or more X-ray diffraction peaks selected from the group consisting of 16.60±0.20 (2Θ), 19.48±0.20 (2Θ), 19.95±0.20 (2Θ), 21.26±0.20 (2Θ), 25.52±0.20 (2Θ), 25.69±0.20 (2Θ), 26.05±0.20 (2Θ), 29.44±0.20 (2Θ), 29.67±0.20 (2Θ), 30.13±0.20 (2Θ), 32.25±0.20 (2Θ), 33.02±0.20 (2Θ), 33.48±0.20 (2Θ), and 38.69±0.20 (2Θ) it additionally has at least one X-ray diffraction peak selected from the group consisting 8.73±0.20 (2Θ), 12.29±0.20 (2Θ), 13.38±0.20 (2Θ), 15.94±0.20 (2Θ), 23.58±0.20 (2Θ), 26.54±0.20 (2Θ), 31.71±0.20 (2Θ), 32.77±0.20 (2Θ), 33.75±0.20 (2Θ), 35.42±0.20 (2Θ), 35.94±0.20 (2Θ), 36.45±0.20 (2Θ), 36.79±0.20 (2Θ), 37.52±0.20 (2Θ), 38.28±0.20 (2Θ), 38.94±0.20 (2Θ), 39.21±0.20 (2Θ) and 39.67±0.20 (2Θ).

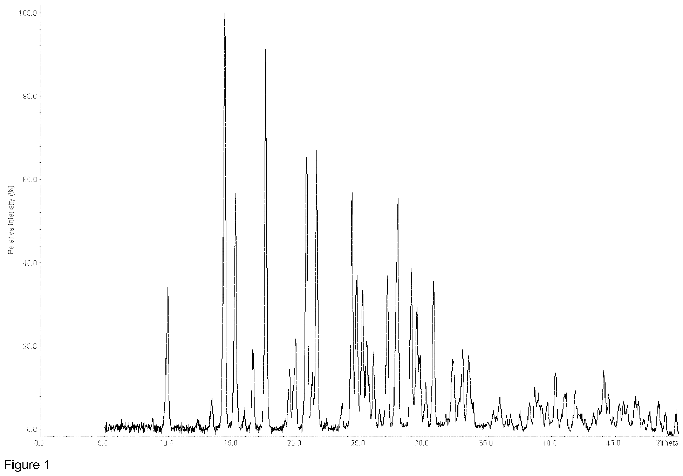

The X-ray powder diffractogramm of the crystalline modification of the salt or cocrystal of hydrobromic acid and (1R,2R)-3-(3-dimethylamino-1-ethyl-2-methyl-propyl)-phenol is shown in figure 1.

Described herein is a method for preparing an inventive salt or cocrystal.

The salt or cocrystal of (a) (1R,2R)-3-(3-dimethylamino-1-ethyl-2-methyl-propyl)-phenol and the at least one acid (b2) may be synthesized by a process including the steps of
- dissolution of a (1R,2R)-3-(3-dimethylamino-1-ethyl-2-methyl-propyl)-phenol compound in a solvent and addition of the acid (b2) or an aqueous solution or suspension or an inert solvent solution or suspension of the acid (b2) preferably dropwise to the solution of the (1R,2R)-3-(3-dimethylamino-1-ethyl-2-methyl-propyl)-phenol compound
   or providing the at least one acid (b2) or an aqueous solution or suspension or an inert solvent solution or suspension of said acid (b2) and addition, preferably dropwise addition of a solution of the (1R,2R)-3-(3-dimethylamino-1-ethyl-2-methylpropyl)-phenol compound dissolved in an inert solvent or water-comprising inert solvent
   or mixing (1R,2R)-3-(3-dimethylamino-1-ethyl-2-methyl-propyl)-phenol compound and the acid (b2) and subsequent addition of an inert solvent or water-comprising inert solvent to said mixture (step 1),
- stirring or keeping the mixture obtained from step 1 at a certain temperature, preferably at room temperature for a certain period of time (step 2),
- optionally cooling the mixture obtained from step 2, preferably to a temperature between 20°C and -40°C (step 3),
- collection of the formed solid, which is - at least when component (a) and component (b2) are employed - usually in a crystalline and/or amorphous form, by filtration and optionally drying the solid (step 4).

The term inert solvent is used herein means that the solvent does not react with the solid components, however the solvent may be incorporated in the salts and/or cocrystals.

The process may optionally contain further steps which independently of one another may be preferably carried out after step 2, such as
- optionally addition of seed crystals,
- optionally evaporation of a part of the solvent,
- optionally addition of a solvent, in which the inventive salt or cocrystal is poorly soluble or insoluble,
- optionally initiation and/or promotion of crystal- and/or co-crystal- precipitation by means of mechanical stimulation.

If a monovalent acid (b2) is employed for the preparation of the inventive salt or cocrystal, the amount of said acid employed is preferably in the range of from 0.4 to 10 mol per mol of component (a), more preferably in the range of from 0.6 to 6 mol, and even more preferably in the range of from 0.8 to 5 mol.

(If a divalent acid (b2) is employed for the preparation of the inventive salt or cocrystal, the amount of said acid employed is preferably in the range of from 0.2 to 10 mol per mol of component (a), more preferably in the range of from 0.3 to 6 mol, and even more preferably in the range of from 0.4 to 5 mol.)

Preferably, the concentration of the acid (b2) to be employed in the aqueous solution or inert solvent solution is in the range of from 0.1 mol/L to saturation, more preferably in the range of from 1 to 20 mol/L, and even more preferably in the range of from 3 to 15 mol/L.

Preferably, the reaction temperature for the preparation of the inventive salt or cocrystal, preferably in step 2, is in the range of from -40°C to 150°C, more preferably in the range of from 0°C to 100°C, even more preferably in the range of from 10°C to 60°C.

Preferably, the reaction time for the preparation of the inventive salt or cocrystal, preferably in step 2, is in the range of from 5 minutes to 48 hours, more preferably in the range of from 10 minutes to 24 hours, even more preferably in the range of from 30 minutes to 12 hours.

The formed solid obtainable in step 4 may be isolated by filtration, centrifugation or a gradient method and may be optionally washed with an inert solvent (preferably an inert solvent employed in the preparation of the salt or cocrystal) as necessary. The isolated solid may be dried under reduced pressure and/or elevated temperature.

Any inert solvents or water-comprising inert solvents suitable for the preparation of the inventive salt or cocrystal may be employed, e.g. any inert solvents or water-comprising inert solvents which do not inhibit the formation of the salt or cocrystal and which allow the starting material employed, i.e. component (a) and (b2) to be dissolved therein to at least an extent necessary for the preparation of the salt or cocrystal.

Preferably, inert solvents or suitable media for the preparation of the inventive salt or cocrystal are selected from the group consisting of aliphatic hydrocarbons, e.g. hexane, pentane, petroleum ether and cyclohexane, aromatic hydrocarbons, e.g. benzene, toluene and xylene, halogenated hydrocarbons, e.g. methylene chloride, chloroform, carbon tetrachloride, dichloroethane, chlorobenzene and dichlorobenzene, ethers, e.g. diethyl ether, diisopropyl ether, dibutyl ether, butyl methyl ether, sec-butyl methyl ether, tert-butyl methyl ether, tetrahydrofuran, dioxane, dimethoxyethane and diethylene glycol dimethyl ether, ketones, e.g. acetone, methyl ethyl ketone, methyl isobutyl ketone and cyclohexanone, esters, e.g. ethyl acetate, propyl acetate and butyl acetate, nitriles, e.g. acetonitrile, propionitrile, butyronitrile and isobutyronitrile, alcohols, e.g. methanol, ethanol, 1-propanol, 2-propanol, 1-butanol, 2-butanol, 2-methyl-1-propanol and 2-methyl-2-propanol, amides, e.g. formamide, dimethylformamide, dimethylacetamide, N-methyl-2-pyrrolidone and hexamethylphosphoric triamide, and any mixtures in any ratio thereof. More preferably, inert solvents suitable for the preparation of the inventive salt or cocrystal are selected from the group consisting of toluene, tert-butyl methyl ether, acetone, ethyl acetate, methanol, ethanol, 1-propanol, 2-propanol and any mixtures in any ratio thereof. Optionally, any of these inert solvents or any combinations thereof may comprise a suitable amount of water.

An aspect of the present invention is a medicament comprising at least one salt or cocrystal as described herein.

In a preferred embodiment, the medicament is a solid medicinal form. However, liquid or pasty medicinal forms are also possible.

Preferably, the medicament is formulated for oral administration. However, pharmaceutical forms that are adapted for other administration routes are also possible, for example buccal, sublingual, transmucosal, rectal, intralumbar, intraperitoneal, transdermal, intravenous, intramuscular, intragluteal, intracutaneous and subcutaneous administration.

Depending upon the formulation, the medicament preferably contains suitable additives and/or excipients. Suitable additives and/or excipients for the purpose of the invention are all substances for achieving galenic formulations known to the person skilled in the art from the prior art. The selection of these excipients and the amounts to use depend upon how the medicinal product is to be administered, i.e. orally, intravenously, intraperitoneally, intradermally, intramusculary, intranasally, buccally or topically.

Suitable for oral administration are preparations in the form of tablets, chewable tablets, dragees, capsules, granules, drops, juices or syrups; suitable for parenteral, topical and inhalative administration are solutions, dispersions, suspensions, easily reconstituted dry preparations and sprays. A further possibility is suppositories for use in the rectum. Use in a depot in dissolved form, a carrier foil or a plaster, optionally with the addition of means to encourage penetration of the skin, are examples of suitable percutaneous administration forms. If administered orally, the most preferred preparation of the inventive medicament is in the form of a tablet.

Examples of excipients and additives for oral administration forms are disintegrants, lubricants, binders, fillers, mould release agents, optionally solvents, flavourings, sugar, in particular carriers, diluents, colorants, antioxidants, etc.

For suppositories, it is possible to use inter alia waxes or fatty acid esters and for parenteral means of application, carriers, preservatives, suspension aids, etc.

Excipients can be for example: water, ethanol, 2-propanol, glycerin, ethylene glycol, propylene glycol, polyethylene glycol, polypropylene glycol, glucose, fructose, lactose, sucrose, dextrose, molasses, starch, modified starch, gelatin, sorbitol, inositol, mannitol, microcrystalline cellulose, methyl cellulose, carboxymethylcellulose, cellulose acetate, shellac, cetyl alcohol, polyvinylpyrrolidone, paraffins, waxes, natural and synthetic rubbers, acacia gum, alginates, dextran, saturated and unsaturated fatty acids, stearic acid, magnesium stearate, zinc stearate, glyceryl stearate, sodium lauryl sulfate, edible oils, sesame oil, coconut oil, groundnut oil, soybean oil, lecithin, sodium lactate, polyoxyethylene and propylene fatty acid ester, sorbitan fatty acid esters, sorbic acid, benzoic acid, citric acid, ascorbic acid, tannic acid, sodium chloride, potassium chloride, magnesium chloride, calcium chloride, magnesium oxide, zinc oxide, silicon dioxide, titanium oxide, titanium dioxide, magnesium sulfate, zinc sulfate, calcium sulfate, potash, calcium phosphate, dicalcium phosphate, potassium bromide, potassium iodide, talc kaolin, pectin, crospovidone, agar and bentonite.

The production of this medicinal product, preparation, medicament and pharmaceutical composition is performed with the aid of means, devices, methods and processes which are well known in the prior art of pharmaceutical formulation, such as those described for example in "Remington's Pharmaceutical Sciences", ed AR Gennaro, 17th edition, Mack Publishing Company, Easton, pa. (1985), in particular in Part 8, Chapters 76 to 93.

For example, for a solid formulation, such as a tablet, the active substance of the medicament can be granulated with a pharmaceutical carrier, e.g. conventional tablet ingredients, such as maize starch, lactose, sucrose, sorbitol, talc, magnesium stearate, dicalcium phosphate or physiologically acceptable rubbers, and pharmaceutical diluents, such as water, for example, to form a solid composition containing the active substance in a homogeneous distribution. Here, a homogeneous distribution should be understood as meaning that the active substance is distributed uniformly throughout the entire composition so that this can be easily divided into equally effective single dose forms, such as tablets, capsules, dragees. The solid composition is then divided into single dose forms. The tablets or pills can also be coated or compounded in some other way in order to produce a dosage form with delayed release. Suitable coating means are inter alia polymers acids and mixtures of polymeric acids with materials such as shellac, for example, cetyl alcohol and/or cellulose acetate.

In a preferred embodiment of the present invention the salt or cocrystal is present in the medicament in immediate release form.

In another preferred embodiment of the present invention the salt or cocrystal is present in the medicament in controlled-release form. For the purpose of the specification, the term "controlled-release form" refers to any type of release form other than an immediate release form and includes e.g. a delayed-release form, a prolonged release from, a sustained release form, a slow release form, an extended release form and the like. These terms are well known to any person skilled in the art as are the means, devices, methods and processes for obtaining such type of release.

"Controlled-release forms" such as delayed-release forms of the inventive salt or cocrystal can, for example, be achieved by retardation by means of a matrix, a coating or release systems with an osmotic action (see e.g. EP-A 1 439 829). However, as stated above, in order to achieve a controlled release of the inventive salt or cocrystal, and thus its component (a), such a formulation is not necessary due to the intrinsic controlled release properties of the inventive salt or cocrystal.

Controlled-release forms of the inventive salt or cocrystal are possible from formulations for oral, rectal or percutaneous administration. Preferably, the medicament is formulated for once-daily administration, for twice-daily administration (*bid*) or for thrice-daily administration, with twice-daily administration (*bid*) being particularly preferred.

The medicament may contain one or more further drugs besides the inventive salt or cocrystal. Preferably, however, the medicament contains at least one, preferably one, salt or cocrystal as the only drug.

The amounts of the inventive salt or cocrystal to be administered to patients vary depending upon the weight of the patient, the method of administration and the severity of the disease and/or pain. The inventive salt or cocrystal may be administered in amounts up to its maximum daily dosage, which is known to those skilled in the art. In a preferred embodiment, the medicament contains the inventive acid addition salt or cocrystal in an amount of 1 to 1000 mg, more preferably 10 to 500 mg, even more preferably 30 to 400 mg, most preferably 40 to 300 mg, as an equivalent dose based on the salt or cocrystal.

The medicament can be provided as a simple tablet and as a coated tablet (e.g. as a film-coated tablet or dragee). The tablets are usually round and biconvex, but oblong shapes are also possible. Granules, spheroids, pellets or microcapsules, which are used to fill sachets or capsules or pressed into disintegrating tablets, are also possible.

Medicaments containing at least 0.001 to 99.999 % of the inventive salt or cocrystal, in particular low, active doses, are preferred in order to avoid side effects. The medicament contains preferably 0.01 % by weight to 99.99 % by weight of the inventive salt or cocrystal, more preferably 0.1 to 90 % by weight, even more preferably 0.5 to 80 % by weight, most preferably 1.0 to 50 % by weight and in particular 5.0 to 20 % by weight. To avoid side effects, it may be advantageous at the start of the treatment to increase the amount of the inventive salt or cocrystal to be administered gradually (titration) to allow the body to become accustomed to the active substance slowly. Preferably, the inventive salt or cocrystal is first administered in a dose which is below the analgesically active dose.

Particularly preferably, the medicament has an oral pharmaceutical form, which is formulated for once daily or twice-daily administration and contains at least one inventive salt or cocrystal in an amount of 10 to 500 mg as an equivalent dose based on the salt or cocrystal.

Another aspect of the present invention is a medicament as defined herein for use in the treatment of pain.

Preferably, the pain is selected from the group consisting of inflammatory pain, neuropathic pain, acute pain, chronic pain, visceral pain, migraine pain and pain associated with cancer.

Preferably, the pain is moderate to strong.

Described herein is a method of treating pain in a mammal, which comprises administering an effective amount of at least one inventive salt or cocrystal to the mammal.

Preferably, the pain is selected from the group consisting of inflammatory pain, neuropathic pain, acute pain, chronic pain, visceral pain, migraine pain and pain associated with cancer. Preferably, the pain is moderate to strong.

Even if the medicaments according to the invention exhibit few side effects only, it may be advantageous, for example, in order to avoid certain types of dependency to use morphine antagonists, in particular naloxone, naltrexone and/or levallorphan, in addition to the inventive salt or cocrystal.

Described herein is a kit comprising a medicament containing an salt or cocrystal and/or dosage forms comprising said salt or cocrystal according to the invention.

The kit is preferably designed for in each case once daily, twice daily or three times daily administration of the medicaments (dosage forms) contained therein.
**Figure 1** shows the X-ray powder diffractogramm of tapentadol hydrobromide.
**Figure 12** shows the dissolution curve of tapentadol hydrobromide (media: SIFsp: diamond, square, triangle; SGFsp: cross, asterisk, circle).

The following examples serve for a further explanation of the invention but should not be construed as restrictive.

### Examples

For the manufacture of the specific salts or cocrystals as described herein below free tapentadol base (referred to in the following as tapentadol) was employed as starting material which can be obtained as described in EP-A 0 693 475.

Reference example **b1-1:** salt or cocrystal of tapentadol and (2S,3S)-dibenzoyl tartaric acid 20 g (0.090 mol) of tapentadol and 16.19 g (0.045 mol) of (+)-(2S,3S) dibenzoyl tartaric acid were dissolved in 400 mL of acetone. The mixture was stirred at room temperature for about 3 hours. The resulting crystalline precipitate was filtered off and dried under reduced pressure (6 mbar) at 40°C (yield 36.1 g, 100%, melting point (DSC): Tₒ= 152.5 °C, Tₚ = 170.7 °C; 9.7 J/g, Tₒ= 185.7 °C, Tₚ = 188.2 °C, 119.1 J/g). ¹H-NMR analysis showed a 2:1-stochiometry of tapentadol and (+)-(2S,3S) dibenzoyl tartaric acid.

Reference example **b1-2:** salt or cocrystal of tapentadol and sebacic acid 125 g (0.56 mol) of tapentadol were dissolved in 700 mL of ethyl acetate. To this solution, 114.21 g (0.56 mol) of sebacic acid were added as a solid in portions. Then, 50 mL of ethyl acetate were added. Subsequently, the resulting suspension was stirred for 15 hours. The resulting crystalline white precipitate was then slowly filtered off and dried at 60°C under reduced pressure (6 mbar) (yield: 232.96 g, 97.37%, melting point (DSC): Tₒ= 77.9 °C, Tₚ = 81.1 °C, 99.5 J/g). ¹H-NMR analysis showed a 1:1-stochiometry of tapentadol and sebacic acid.

Reference example **b1-3:** salt or cocrystal of tapentadol and 1-hydroxy-2-naphthoic acid 120 g (0.54 mol) of tapentadol were dissolved in 700 mL of 2-propanol. To this solution, 102.02 g (0.54 mol) of 1-hydroxy-2-naphthoic acid were added as a solid in portions. Then, 100 mL of 2-propanol were added. After complete addition, the resulting suspension was stirred for 18 hours. The crystallized brownish precipitate was then filtered off and dried at 50°C under reduced pressure (6 mbar) (yield: 199.43 g, 89.83%, melting point (DSC): Tₒ= 114.9 °C, Tₚ = 122.7, °C, 1.0 J/g; Tₒ= 157.6°C, Tₚ = 159.9 °C, 78.3 J/g). ¹H-NMR analysis showed a 1:1-stochiometry of tapentadol and 1-hydroxy-2-naphthoic acid.

Reference example **b1-4:** salt or cocrystal of tapentadol and embonic acid
**b1-4-1:**
   8 g (0.036 mol) of tapentadol and 14.037 g of embonic acid (0.036 mol) were suspended in 300 mL of 2-propanol. After addition, the resulting suspension was stirred for 19 hours. The crystallized yellow precipitate was then slowly filtered off and dried at 50°C under reduced pressure (6 mbar) for 7 hours (yield: 20.86 g, 94.7%, melting point (DSC): Tₒ= 114.7 °C, Tₚ = 122.0, °C; 1.5 J/g; Tₒ= 214.8°C, Tₚ = 219.1 °C, 58.0 J/g). ¹H-NMR analysis showed a 1:1-stochiometry of tapentadol and embonic acid.
**b1-4-2:**
   877.32 mg of embonic acid (0.00226 mol) were suspended in 50 mL of ethanol. To this mixture, 1 g (0.0045 mol) of tapentadol in 5 mL of ethanol was added. After addition, the resulting suspension was stirred for 1 hour. The crystallized white precipitate was then slowly filtered off and dried at 40°C under reduced pressure (6 mbar) for 3 days (yield: 1.564 g, 83.3%, melting point (DSC): Tₒ= 222.6 °C, Tₚ = 226.5, °C, 96.7 J/g). ¹H-NMR analysis showed a 2:1-stochiometry of tapentadol and embonic acid (hemi-embonate).
**b1-4-3:**
   110 g (0.496 mol) of tapentadol were dissolved in 3 L of 2-propanol. To this solution, 193.0 g (0.496 mol) of embonic acid were added as a solid to the resulting solution in portions. Then, 1 L of 2-propanol was added. After addition, the resulting suspension was stirred for 24 hours. The crystallized yellow precipitate was then slowly filtered off and dried at 60°C under reduced pressure (6 mbar) (yield: 298.14 g, 98.36%). ¹H-NMR analysis showed a 1:0.9-stochiometry of tapentadol and embonic acid. To remove the excessive free tapentadol base, the solid was suspended in 1.7 L of ethyl acetate and stirred. After 3 hours, the crystallized yellow precipitate was then slowly filtered off and dried at 60°C under reduced pressure (3 mbar) (yield: 288.43 g, 95.16%; melting point (DSC): Tₒ= 217.3 °C, Tₚ = 220.1, °C; 57.2 J/g. Weight loss (TGA, range 31 to 171 °C): less then 0.1 %. ¹H-NMR analysis showed a 1:1-stochiometry of tapentadol and embonic acid. According to XRPD analysis polymorph A was obtained.
**b1-4-4:**
   501.32 mg of tapentadol and 878.83 mg of embonic acid were given into a PLS-vial and suspended subsequently in 30 mL of tetrahydrofuran. The resulting mixture was then vortexed at 30 °C and 400 rpm overnight. Afterwards the solvent was evaporated off to yield a yellow oil that was left in the refrigerator for crystallization. After 7 days a very rigid yellow solid was obtained, which was analysed via ¹H-NMR spectroscopy. It was found that the sample contained approximately 8% tetrahydrofuran. The solid was therefore dried at 40 °C and approximately 7 mbar for 16 hours. ¹H-NMR analysis of the dried solid showed a 1:1-stochiometry of tapentadol and embonic acid with approximately 5% tetrahydrofuran. The product showed a melting point (DSC) of Tₒ=35.3°C; Tₚ=58.0°C; 1.4 J/g; Tₒ=125.2°C; Tₚ=130.1°C; 46.7 J/g Tₒ=208.6°C; Tₚ=217.2°C; 41.0 J/g. Weight loss (TGA; range 53 to 167°C): -5.7 %; Tₒ=119.41°C; Tₒ=205.50°C. According to XRPD analysis polymorph B was obtained.

Reference example **b1-5:** salt or cocrystal of tapentadol and nitric acid 20 g (0.090 mol) of tapentadol were dissolved in 70 mL of acetone. To this solution were added 6.30 mL of nitric acid at room temperature in 100 µL-portions. The solvent was evaporated off under reduced pressure and the resulting yellow oil was stirred at room temperature for 16 h. After 16, part of the oily material was crystallized. 50 mL of ethyl acetate were added and the remaining oily material also crystallized within minutes. 40 mL of the solvent was evaporated. 60 mL of acetone were added. The resulting suspension was refluxed for 10 minutes at 50°C. 100 mL of acetone were added and the mixture was again refluxed for 10 minutes at 50°C. 40 mL of the solvent was evaporated and the resulting mixture stirred for 1.5 hours. The crystallized precipitate was then filtered off and dried under reduced pressure (6 mbar) at 40°C (yield 21.32 g, 83%, melting point (DSC): Tₒ= 86.9 °C, Tₚ = 88.2 °C, 0.4 J/g; Tₒ= 119.7 °C, Tₚ = 121.3 °C, 119.3 J/g). ¹H-NMR analysis showed a 1:1-stochiometry of tapentadol and nitric acid.

Reference example **b2-1a:** salt or cocrystal of tapentadol and nicotinic acid To 20 g (0.090 mol) of tapentadol and 11.126 g (0.090 mol) of nicotinic acid 120 mL of THF were added, resulting in a grey-colored suspension. The precipitate of said suspension was then filtered off. The filtrate was kept at room temperature and the solvent was slowly evaporated off. After 23 hours a green-colored oil was obtained to which 150 mL of n-hexane were added. The mixture of n-hexane and the oily material was then stirred for 1 hour at 50°C resulting in a mixture of n-hexane and a white-colored oily material, which began to crystallize slowly. The resulting mixture was stirred for another 3 hours at 50°C until about half of the solvent had been evaporated off. The mixture was then kept at room temperature for another 23 hours. The precipitate was filtered off, washed with 40 mL of n-hexane and dried under reduced pressure (6 mbar) at 40°C yielding a grey-colored solid (28.73 g, 92.3%). ¹H-NMR analysis showed a 1:0.9-stochiometry of tapentadol and nicotinic acid. The obtained product was a mixture of two polymorphous forms of the salt or cocrystal and additionally contained free tapentadol base. In order to obtain a pure crystalline form of the salt or cocrystal 26.309 g of said product were suspended in 350 mL of diethyl ether and stirred at room temperature. The mixture was kept at room temperature for 4 days and the resulting crystallized precipitate was filtered off and dried at 40°C under reduced pressure (yield: 24.304 g, 92.38%, melting point (DSC): Tₒ= 66.2 °C, Tₚ = 72.7 °C, 6.7 J/g; Tₒ= 100.6 °C, Tₚ = 104.2 °C, 79.9 J/g). ¹H-NMR analysis showed a 1:1-stochiometry of tapentadol and nicotinic acid. According to XRPD a mixture of two polymorphs was obtained.

Reference example **b2-1b:** salt or cocrystal of tapentadol and nicotinic acid 500 mg (0.0023 mol) of tapentadol were dissolved in 4 mL acetone. 278 mg nicotinic acid were dissoled in 22 mL tetrahydrofuran at 55 °C and added to the solution of tapentadol

The solvent was evaporated over night at 23°C with a flow of air. After 18 hours 6 mL of hexane were added to the yellowish oil and scratched mechanically with a spatula giving an white to off white oil. Again the solvent was evaporated at 23° C by application of a flow of air for one hour giving a white to off white oil. The said oil was dried at 40°C and under reduced pressure (6 mbar) for two hours yielding an almost colorless to colorless oil. After cooling to 23°C the said oil remained colorless and sticky. Furthermore 2 mL of hexane were added to the oil and the mixture was scratched with a spatula. Over 4 days the solvent was slowly evaporated off yielding white to grey crystalline material (agglomerates). Again 6 mL of hexane were added, agglomerates were rduced in size mechanically with a spatula. After 24 hours of vortexing at 21°C the grey solid was filtered off and dried at 40°C under reduced pressure for 1 hour yielding grey to white crystals (yield: 749 mg, 96.3%, melting point (DSC): Tₒ= 70.5 °C, Tₚ = 76.2 °C, 103.3 J/g; weight loss (TGA, range: 27 to 171 °C): -5.9 %). ¹H-NMR analysis showed a 1:1-stochiometry of tapentadol and nicotinic acid. According to XRPD analysis polymorph B¹ was obtained.

Reference example **b2-1c:** salt or cocrystal of tapentadol and nicotinic acid 120.0 g tapentadol and 66.744 g nicotinic acid (1 equivalent) were given into a 1L flask and 700 ml tetrahydrofuran were added subsequently. A grey-brownish suspension was obtained comprising a little amount of undissolved white solid (which was analysed to be nicotinic acid). The suspension was stirred at room temperature for 17 hours and still contained undissolved nicotinic acid. 100 mL of tetrahydrofurane were added, but the nicotinic acid still did not dissolve completely. Within 10 minutes the suspension was heated to its boiling point with a heat gun. After 2 minutes a brownish solution was obtained, which was cooled to room temperature and evaporated off to a volume of approximately 600 mL in an airstream. Subsequently, the solution was evaporated to dryness using a water bath at 40 °C. A viscous oil was obtained that could not be dried completely. The oil was dried in vacuo at 100 mbar. Subsequently 500 mL n-hexane were added and the oil was distributed on the wall of the flask by means of a spatula (white oil on the wall). The solvent was evaporated off overnight in an airstream. After 17 hours a white solid was obtained on the wall of the flask and a greenish solid was obtained at the bottom of the flask with white crystals on its surface. The very hard residue was reduced to smaller parts with a spatula. Subsequent to filtration the white solid and the greyish-white solid were pestled and combined with the filtrate. N-Hexane was added to this suspension (to a total volume of 700 mL) and the suspension was stirred at room temperature. After 17 hours at room temperature the solid was filtered off, sucked dry and then further dried at 40 °C and approximately 3 mbar. The filtrate was discarded. After 3 hours the solid was pulverized and dried further to yield 185.406 g (99.28 of theoretic yield) of solid product. ¹H-NMR analysis showed a 1:1-stochiometry of tapentadol and nicotinic acid. XRPD showed that only one polymorphic form (polymorph A¹) was obtained. Melting point (DSC): Tₒ=40.16 °C; Tₚ=54.65 °C; 1.39 J/g; Tₒ=102.90 °C; Tₚ=105.28 °C; 97.93 J/g, TG yielded -0.19 %(29.76-135,97 °C); Tₒ=100,22°C; weight loss (TGA, range: 25 to 148 °C): less then 0.3 %).

### Example b2-2: salt or cocrystal of tapentadol and hydrobromic acid

20 g (0.090 mol) of tapentadol were dissolved in 400 mL of ethyl acetate. To this solution, 10.22 mL (0.090 mol) of hydrobromic acid (48%) were added dropwise. After addition, the resulting suspension was stirred for another 3 hours. The crystallized precipitate was filtered off and dried at 40°C under reduced pressure (6 mbar) (yield: 25.96 g, 95.0%, 1:1-stochiometry of tapentadol and hydrobromic acid; melting point (DSC): Tₒ= 184.9 °C, Tₚ = 185.4 °C, 110.4 J/g).

### Reference example b2-3: salt or cocrystal of tapentadol and sulfuric acid

### b2-3-1:

500 mg (2.26 mmol) of tapentadol were dissolved in 5 mL of acetone. To this solution, 125 µL of sulfuric acid (96%) were added. The mixture was stirred for 30 minutes at room temperature. The solvent was then evaporated off at 50°C. To the remaining oily yellowish residue 5 mL of cyclohexane were added and the resulting mixture was stirred for 30 minutes. Subsequently, the mixture was kept at room temperature for 8 days. The oily residue was then dissolved in 5 mL of ethanol. The solvent was partly evaporated off (about 4 mL) and 5 mL of cyclohexane were added again and the resulting mixture was stirred for 30 minutes and kept for 6 days at room temperature yielding an oily material. The solvent was slowly evaporated off at room temperature and under normal pressure. After 7 days the oily material had crystallized. 5 mL of n-hexane were added resulting in a white suspension. The resulting suspension was stirred for another 30 minutes and kept at room temperature for another 6 days. The precipitated crystallized solid was the filtered off and dried in vacuo (yield: 678.73 mg, 94.1%, melting point (DSC): Tₒ= 63.1 °C, Tₚ = 65.1 °C, 14.1 J/g; Tₒ= 72.6 °C, Tₚ = 77.5 °C, 83.8 J/g).

### b2-3-2:

44.3 mg (0.2 mmol) of tapentadol were dissolved in 1 mL of ethanol. To this solution, 10.7 µL (0.2 mmol) of sulfuric acid (96%) were added. The solvent was then evaporated off at 50°C within 15 minutes. The remaining colorless oil was kept for 13 days at room temperature. After 13 days the oil had completely crystallized (yield: 62 mg, 97.0%). ¹H-NMR analysis showed a 1:1-stochiometry of tapentadol and sulfuric acid (sulfate salt or cocrystal of tapentadol).

### Reference example b2-4: salt or cocrystal of tapentadol and fumaric acid

### b2-4-1:

20 g (0.090 mol) of tapentadol and 5.244 g (0.045 mol) of fumaric acid were homogenized in a 250 mL-flask. 200 mL of 2-propanol were added and the resulting mixture was stirred at 40°C for about 10 minutes. The solvent was then partly evaporated off under reduced pressure. During evaporation, a white solid crystallized. 100 mL of ethyl acetate were added and the resulting suspension was stirred for another 30 minutes at room temperature. The resulting mixture was then kept at room temperature for 7 days. The precipitated crystallized solid was filtered off, washed with 20 mL of ethyl acetate. The solvents were evaporated under reduced pressure and the remaining solid was dried in vacuo (yield: 24.765 g, 98.1%; melting point (DSC): Tₒ= 134.0 °C, Tₚ = 135.7 °C, 125.1 J/g). ¹H-NMR analysis showed a 2:1-stochiometry of tapentadol and fumaric acid (hemi fumarate).

### b2-4-2:

To 31.464 g (0.271 mol) of fumaric acid was added 1 L of acetone. The resulting mixture was stirred for 30 minutes. To this suspension, a solution of 120 g (0.542 mol) of tapentadol in 500 mL of acetone was added dropwise within 50 minutes. After addition of about 120 mL of said solution, the starting material is completely dissolved. After addition of about 150 mL of said solution, a white solid begins to precipitate. After addition of the complete solution of tapentadol, the solid had turned into an oily material. The resulting mixture was stirred at room temperature for another 10 minutes. After about 10 minutes the oily material started to crystallize. The resulting mixture was then kept at room temperature for 19 hours. The crystallized solid was filtered off and dried under reduced pressure (6 mbar) at 40°C (yield: 147.24 g, 97.2%; melting point (DSC): Tₒ= 134.0 °C, Tₚ = 135.1 °C, 127.6 J/g). ¹H-NMR analysis showed a 2:1-stochiometry of tapentadol and fumaric acid (hemi fumarate).

### Reference example b2-5: salt or cocrystal of tapentadol and malonic acid

20 g (0.090 mol) of tapentadol and 9.403 g of malonic acid (0.090 mol) were dissolved in 300 mL of ethyl acetate. After addition, the resulting suspension was stirred for 18 hours. The crystallized precipitate was then filtered off and dried at 40°C under reduced pressure (6 mbar) (yield: 28.797 g, 97.9%,melting point (DSC): Tₒ= 109.8 °C, Tₚ = 111.4 °C, 86.9 J/g). ¹H-NMR analysis showed a 1:1-stochiometry of tapentadol and malonic acid.

### Analysis - NMR

Structural characterization was conducted using ¹H-NMR spectroscopy at 400 MHz.

| | |
|---|---|
| Instrument: | Bruker Avance II 400 equipped with a BBO (Broad Band Observ) probe. |
| Solvent: | D6-dimethyl sulfoxide 99.9 atom-%, 0.1 v/v-% TMS (Aldrich) or Chloroform-d 99.8 atom-%, 0.1 v/v-% TMS (Aldrich) |
| Temperature: | 303 (± 1) K |

The TMS (Tetramethylsilane) signal was used as an internal reference at 0.00 ppm.

### Analysis - XRPD

X-ray powder diffractometry (XRPD, X-ray powder diffraction):
XRPD investigations were conducted with a STOE Stadi P X-ray powder diffractometer in transmission geometry, using CuKα₁ radiation made monochromatic by means of a germanium single crystal.

### Stoe Stadi P

| | |
|---|---|
| Diffractometer : | Transmission |
| Monochromator: | Curved, Germanium (111) |
| Wavelength: | Cu Kα |
| Detektor: | Linear PSD |
| Scan Modus: | Transmission / Moving PSD / Fixed omega |
| Scan Type: | 2Theta: Omega (2Theta: 2°-50°, step 0.5°, Omega 1°-25°, step 0.25°, time/step 30 s) |

D-spacings may be calculated using Bragg's law from the 2θ values, based on a wavelength of 1.54060 Å. As a general rule the 2θ values have an error rate of ±0.2° in 2Θ. The experimental error for the d-spacing values therefore depends on the position of the line (of the peak).

### Analysis - DSC

Differential Scanning Calorimetry (DSC) measurements were performed with a Mettler-Toledo DSC 821 Differenzthermoanalyse.

Unless otherwise specified, the samples were weighed in a aluminium crucible (lid with pinhole) and measured in a nitrogen flow. Generally DSC investigations were performed in a temperature range of 25 to about 200 °C or resp. 350 °C with a heating rate of 10 °C/min. The temperatures specified in relation to DSC analyses are, unless otherwise specified, the temperatures of the peak maxima (peak temperature T_{P}). Onset temperatures of peaks are indicated by T_{O}. Specific heat is given in J/g.

### Analysis - TGA

Thermogravimetric investigations were performed with a Mettler-Toledo TGA/SDTA851. Unless otherwise specified, the samples were weighed in a aluminium crucible and measured in nitrogen flow. Generally TGA investigations were performed in a temperature range of 25 to about 200 °C or resp. 350 °C with a heating rate of 10 °C/min.

### Analysis - Dissolution

Dissolution experiments were performed with a Sotax AT7 smart using UV-detection (Photometer Perkin Elmer). Vessels equipped with a Wood apparatus (for intrinsic dissolution) were used.

The tablets with a surface of 0.5 cm² (diameter 8 mm) were made by applying press capacity of 200 N (200 kg applied for 1 min) using about 100 mg of the particular substance.

Dissolutions were performed in the following dissolution media:
- SIFsp; intestinal fluid without pancreas powder (pancreatin), pH 6,8 (according to Ph. Eur.);
- SGFsp; gastric fluid without pancreas powder (pancreatin), pH 1.2 (according to Ph. Eur.);

Every single dissolution experiment was made in a volume of 900 ± 5 mL (temperature of media: 37 ± 1 °C, rotation speed: 100 ± 5 rpm).

The content (percentage of base released; % base released) was detected using UV-spectroscopy, wavelength: 271 nm, path length (cuvette): 10 mm. Samples were drawn manually.

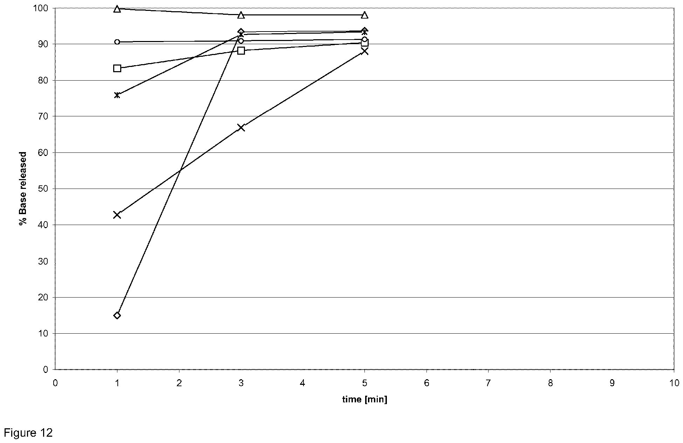

The dissolution curves are shown in figures 12-21b.

In the following tables some of the results of further characterizations of some of the inventive salts or cocrystals, obtained from different samples, are given in detail
*A) Hemi salt or hemi cocrystal of (2S,3S)-Di-benzoyl tartaric acid and tapentadol*

| | **A-1** | A-2 | A-3 |
|---|---|---|---|
| **¹H-NMR** | 1:0.5 | 1:0.5 | 1:0.5 |
| **XRPD** | crystalline | crystalline | crystalline |
| **DSC** | To 152.5 °C | To=190.0 °C | To=188.5 °C |
| | Tp 170.7 °C | Tp=191.2 °C | Tp=190.1°C |
| | 9.7 J/g | 137.8 J/g | 126.6 J/g |
| | To 185.7 °C | | |
| | Tp 188.2 °C | | |
| | 119.1J/g | | |
| **TGA** | no weight loss up to 140 °C | no weight loss up to 160 °C | no weight loss up to 160 °C |

*B) Salt or cocrystal of sebacid acid and tapentadol*

| | **B-1** | B-2 | B-3 |
|---|---|---|---|
| **¹H-NMR** | 1:1 | 1:1 | 1:1 |
| **XRPD** | crystalline | crystalline | crystalline |
| **DSC** | To 78.6 °C | To=76.6 °C | To=79.0 °C |
| | Tp 82.3 °C | Tp=80.6 °C | Tp=82.2 °C |
| | 104.5 J/g | 69.8 J/g | 102.5 J/g |
| **TGA** | no weight loss up to 140 °C | no weight loss up to 105 °C | no weight loss up to 145 °C |

*C) Salt or cocrystal of 1-hydroxy-2-naphthoic acid and tapentadol*

| | **C-1** | C-2 |
|---|---|---|
| **¹H-NMR** | 1:1 | 1:1 |
| **XRPD** | crystalline | crystalline |
| **DSC** | To 112.4 °C | To=157.3 °C |
| | Tp 120.7 °C | Tp=160.3 °C |
| | 1,1 J/g | 82.6 J/g °C |
| | To 153.4 °C | |
| | Tp 157.4 °C | |
| | 69.1J/g | |
| **TGA** | no weight loss up to 125 °C | no weight loss up to 130 °C |

*D) Salt or cocrystal of embonic acid and tapentadol*

| | **D-1** | D-2 | D-3 |
|---|---|---|---|
| **¹H-NMR** | 1:1 | 1:1 | 1:1 |
| **XRPD** | crystalline | crystalline | crystalline |
| **DSC** | To 127.2 °C | To=221.7 °C | To=217.2 °C |
| | Tp 130.6 °C | Tp=225.0 °C | Tp=220.0 °C |
| | 38,7 J/g | 56.1 J/g | 62.9 J/g |
| | To 141.3 °C | | |
| | Tp 149.2 °C | | |
| | -6,0 J/g | | |
| | To 209.9 °C | | |
| | Tp 218.9 °C | | |
| | 46.0 J/g | | |
| **TGA** | -7.1 % (90 °C- 157°C) | no weight loss up to 170 °C | no weight loss up to 160 °C |

*E) Salt or cocrystal of malonic acid and tapentadol*

| | **E-1** |
|---|---|
| **¹H-NMR** | 1:1 |
| **XRPD** | crystalline |
| **DSC** | To 109.8 °C |
| | Tp 111.4 °C |
| | 86.9 J/g |
| **TGA** | no weight loss up to 115 °C |

*F) Salt or cocrystal of hydrobromic acid and tapentadol*

| | **F-1** |
|---|---|
| **¹H-NMR** | 1:1 |
| **XRPD** | crystalline |
| **DSC** | To 184.9 °C |
| | Tp 185.4 °C |
| | 110.4 J/g |
| **TGA** | no weight loss up to 195 °C |

*G) Salt or cocrystal of nicotinic acid and tapentadol*

| | **G-1** | G-2 | G-3 | G-4 |
|---|---|---|---|---|
| **¹H-NMR** | 1:1 | 1:1 | 1:1 | 1:1 |
| **XRPD** | crystalline | crystalline | crystalline | crystalline |
| **DSC** | To 69.4 °C | To 103.1 °C | To=102.7 °C | To=48.8 °C |
| | Tp 74.4 °C | Tp 105.4 °C | Tp=105.2 °C | Tp=65.3 °C |
| | 100.9 J/g | 87.0 J/g | 100.9 J/g | 8.3 J/g |
| | | | | To=103.3°C |
| | | | | Tp=105.2°C |
| | | | | 99.6 J/g |
| **TGA** | -6.4 % (28°C - 171°C) | no weight loss up to 135 °C | no weight loss up to 150 °C | no weight loss up to 135 °C |

*H) Salt or cocrystal of nitric acid and tapentadol*

| | **H-1** |
|---|---|
| **¹H-NMR** | 1:1 |
| **XRPD** | crystalline |
| **DSC** | To 86.9 °C |
| | Tp 88.2°C |
| | 0.37 J/g |
| | To 119.7 °C |
| | Tp 121.3 °C |
| | 119.3 J/g |
| **TGA** | no weight loss up to 170 °C |

*I) Hemi salt or hemi cocrystal of fumaric acid and tapentadol*

| | **I-1** | I-2 | I-3 |
|---|---|---|---|
| **¹H-NMR** | 1:0.5 | 1:0.5 | 1:0.5 |
| **XRPD** | crystalline | crystalline | crystalline |
| **DSC** | To 134.0 °C | To=131.8 °C | To=133.9 °C |
| | Tp 135.7 °C | Tp=133.9 °C | Tp=135.2 °C |
| | 125.1 J/g | 105.4 J/g | 124.9 J/g |
| **TGA** | no weight loss up to 131 °C | no weight loss up to 130 °C | no weight loss up to 130 °C |

*J) Salt or cocrystal of saccharin and tapentadol*

| | **J-1** |
|---|---|
| **¹H-NMR** | 1:1 |
| **XRPD** | crystalline |
| **DSC** | To=110.3 °C |
| | Tp=112.8 °C |
| | 77.3 J/g |
| **TGA** | no weight loss up to 105 °C |

## Claims

1. A medicament comprising at least one salt or cocrystal of
(a) (1R,2R)-3-(3-dimethylamino-1-ethyl-2-methyl-propyl)-phenol, and
(b2) hydrogen bromide,
wherein the salt or cocrystal of component (a) and component (b2) is present in crystalline and/or amorphous form
for use in the treatment of pain.

2. The medicament for use according to claim 1, **characterized in that** the stoichiometry of component (a) and (b2) is within the range of from 1:0.4 to 1:2.1, preferably within the range of from 1:0.4 to 1:1.

3. The medicament for use according to any of the proceeding claims, wherein the salt or cocrystal is a crystalline modification of (1R,2R)-3-(3-dimethylamino-1-ethyl-2-methyl-propyl)-phenol and hydrobromic acid having at least one or more X-ray diffraction peaks selected from the group consisting of 9.93±0.20 (2Θ), 14.39±0.20 (2Θ), 15.22±0.20 (2Θ), 17.60±0.20 (2Θ), 20.81±0.20 (2Θ), 21.61±0.20 (2Θ), 24.37±0.20 (2Θ), 24.73±0.20 (2Θ), 25.19±0.20 (2Θ), 27.14±0.20 (2Θ), 27.94±0.20 (2Θ), 29.00±0.20 (2Θ) and 30.75±0.20 (2Θ).

4. The medicament for use according to any of the proceeding claims, **characterized in that** it is solid, preferably in the form of a tablet.

5. The medicament for use according to any of the proceeding claims, **characterized in that** the medicament is formulated for oral administration.

6. The medicament for use according to any of the proceeding claims, which contains 0.01 % by weight to 99.99 % by weight of the at least one salt or cocrystal, preferably 0.1 to 90 % by weight, more preferably 0.5 to 80 % by weight, even more preferably 1.0 to 50 % by weight and most preferably 5.0 to 20 % by weight.

7. The medicament for use according to any of the proceeding claims, **characterized in that** the pain is selected from the group consisting of inflammatory pain, neuropathic pain, acute pain, chronic pain, visceral pain, migraine pain and pain associated with cancer.

## Patentansprüche

1. Medikament, umfassend mindestens ein Salz oder einen Kokristall aus
(a) (1R,2R)-3-(3-Dimethylamino-1-ethyl-2-methylpropyl)phenol und
(b2) Bromwasserstoff,
wobei das Salz oder der Kokristall aus Komponente (a) und Komponente (b2) in kristalliner und/oder amorpher Form zur Verwendung bei der Schmerzbehandlung vorliegt.

2. Medikament zur Verwendung nach Anspruch 1, **dadurch gekennzeichnet, dass** die Stöchiometrie von Komponente (a) und (b2) innerhalb des Bereichs von 1:0,4 bis 1:2,1, vorzugsweise innerhalb des Bereichs von 1:0,4 bis 1:1, liegt.

3. Medikament zur Verwendung nach einem der vorhergehenden Ansprüche, wobei das Salz oder der Kokristall eine kristalline Modifikation von (1R,2R)-3-(3-Dimethylamino-1-ethyl-2-methylpropyl)phenol und Bromwasserstoffsäure ist, die mindestens einen oder mehrere Röntgenbeugungs-Peaks aufweist, ausgewählt aus der Gruppe, bestehend aus 9,93 ± 0,20 (2Θ), 14,39 ± 0,20 (2Θ), 15,22 ± 0,20 (2Θ), 17,60 ± 0,20 (2Θ), 20,81 ± 0,20 (2Θ), 21,61 ± 0,20 (2Θ), 24,37 ± 0,20 (2Θ), 24,73 ± 0,20 (2Θ), 25,19 ± 0,20 (2Θ), 27,14 ± 0,20 (2Θ), 27,94 ± 0,20 (2Θ), 29,00 ± 0,20 (2Θ) und 30,75 ± 0,20 (2Θ).

4. Medikament zur Verwendung nach einem der vorhergehenden Ansprüche, **dadurch gekennzeichnet, dass** es fest ist, vorzugsweise in Form einer Tablette.

5. Medikament zur Verwendung nach einem der vorhergehenden Ansprüche, **dadurch gekennzeichnet, dass** das Medikament zur oralen Verabreichung formuliert ist.

6. Medikament zur Verwendung nach einem der vorhergehenden Ansprüche, das 0,01 Gew.-% bis 99,99 Gew.-% des mindestens einen Salzes oder Kokristalls, vorzugsweise 0,1 bis 90 Gew.-%, bevorzugter 0,5 bis 80 Gew.-%, noch bevorzugter 1,0 bis 50 Gew.-% und am meisten bevorzugt 5,0 bis 20 Gew.-%, enthält.

7. Medikament zur Verwendung nach einem der vorhergehenden Ansprüche, **dadurch gekennzeichnet, dass** der Schmerz ausgewählt ist aus der Gruppe, bestehend aus inflammatorischem Schmerz, neuropathischem Schmerz, akutem Schmerz, chronischem Schmerz, viszeralem Schmerz, Migräneschmerz und Krebs zugeordnetem Schmerz.

## Revendications

1. Médicament comprenant au moins un sel ou cocristal de
(a) (1R,2R)-3-(3-diméthylamino-1-éthyl-2-méthyl-propyl)-phénol, et
(b2) bromure d'hydrogène,
dans lequel le sel ou le cocristal du composant (a) et du composant (b2) est présent sous forme cristalline et/ou amorphe pour une utilisation dans le traitement de la douleur.

2. Médicament pour une utilisation selon la revendication 1, **caractérisé en ce que** la stœchiométrie des composants (a) et (b2) est comprise dans la plage de 1:0,4 et 1:2,1, de préférence dans la plage de 1:0,4 et 1:1.

3. Médicament pour une utilisation selon l'une quelconque des revendications précédentes, dans lequel le sel ou le cocristal est une modification cristalline du (1R,2R)-3-(3-diméthylamino-1-éthyl-2-méthyl-propyl)-phénol et de l'acide bromhydrique ayant au moins un ou plusieurs pics de diffraction des rayons X sélectionnés parmi le groupe constitué de 9,93±0,20 (2Θ), 14,39±0,20 (2Θ), 15,22±0,20 (2Θ), 17,60±0,20 (2Θ), 20,81±0,20 (2Θ), 21,61±0,20 (2Θ), 24,37±0,20 (2Θ), 24,73±0,20 (2Θ), 25,19±0,20 (2Θ). 27,14 ± 0,20 (2Θ), 27,94 ± 0,20 (2Θ), 29,00 ± 0,20 (2Θ) et 30,75 ± 0,20 (2Θ).

4. Médicament pour une utilisation selon l'une quelconque des revendications précédentes, **caractérisé en ce qu'**il est solide, de préférence sous forme de comprimé.

5. Médicament pour une utilisation selon l'une quelconque des revendications précédentes, **caractérisé en ce que** le médicament est formulé pour une administration orale.

6. Médicament pour une utilisation selon l'une quelconque des revendications précédentes, qui contient de 0,01 % en poids à 99,99 % en poids de l'au moins un sel ou cocristal, de préférence de 0,1 % à 90 % en poids, de manière davantage préférée de 0,5 % à 80 % en poids, de manière davantage préférée encore de 1,0 % à 50 % en poids et de manière préférée entre toutes de 5,0 % à 20 % en poids.

7. Médicament pour une utilisation selon l'une quelconque des revendications précédentes, **caractérisé en ce que** la douleur est sélectionnée parmi le groupe constitué de la douleur inflammatoire, de la douleur neuropathique, de la douleur aiguë, de la douleur chronique, de la douleur viscérale, de la douleur migraineuse et de la douleur associée au cancer.
